(19) Europäisches Patentamt
European Patent Office
Office européen des brevets

(11) Veröffentlichungsnummer: **0 379 953 A2**

# (12) EUROPÄISCHE PATENTANMELDUNG

(21) Anmeldenummer: **90100888.8**

(22) Anmeldetag: **17.01.90**

(51) Int. Cl.[5]: **C07D 413/04, C07D 417/04, C07D 405/04, A61K 31/495, A61K 31/535, A61K 31/54**

Patentansprüche für folgende Vertragsstaaten: ES + GR

(30) Priorität: **21.01.89 DE 3901720**

(43) Veröffentlichungstag der Anmeldung: **01.08.90 Patentblatt 90/31**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Weidmann, Klaus, Dr.**
**Talweg 11**
**D-6242 Kronberg/Taunus(DE)**
Erfinder: **Englert,Heinrich Christian, Dr.**
**Stormstrasse 13**
**D-6238 Hofheim am Taunus(DE)**
Erfinder: **Schölkens, Bernward, Prof. Dr.**
**Hölderlinstrasse 62**
**D-6233 Kelkheim (Taunus)(DE)**
Erfinder: **Bickel, Martin, Dr.**
**Mittelstedter Weg 3**
**D-6380 Bad Homburg(DE)**

(54) **Substituierte Benzo (b) pyrane, Verfahren zu ihrer Herstellung, ihre Verwendung sowie pharmazeutische Präparate auf Basis dieser Verbindungen.**

(57) Benzo[b]pyran-Derivate der Formel I

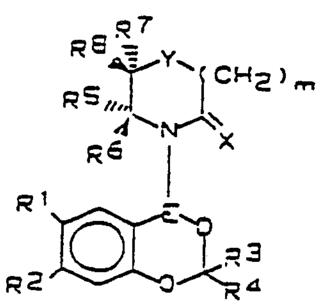

I,

mit E-D gleich CH-CHOH oder C = CH;
X gleich Sauerstoff oder Schwefel;
Y gleich Sauerstoff, Schwefel, SO, $SO_2$ oder $NR^9$;
$R^1$ gleich CN, $NO_2$, Hal, Alkoxycarbonyl, $SO_{1-2}$-Alkyl, $SO_{1-2}$-Aryl;
$R^2$ gleich H, OH, Alkoxy, Alkyl, Hal, $NR^{10}R^{11}$;
$R^3$/$R^4$ gleich Alkyl;
$R^5$/$R^6$ gleich Alkyl, $(CH_2)_{1-6}$COO-Alkyl, $(CH_2)_{1-6}CONR^{10}R^{11}$, $(CH_2)_{0-6}$COOH, $(CH_2)_{1-6}$CO-Alkyl, $CO_2$-Alkyl, Alkylmercaptoalkyl, Alkylsulf(i)(o)nyl, Hydroxylalkyl, Mercaptoalkyl, Aminoalkyl, N-(Di)-Alkylaminoalkyl, $(CH_2)_f$Ar mit f gleich null-3;
$R^7$/$R^8$ gleich Wasserstoff, Alkyl, Phenyl,
m gleich null-2

EP 0 379 953 A2

sind hervorragende Antihypertensiva und Spasmolytika. Herstellungsverfahren und Anwendung werden beschrieben.

## Substituierte Benzo[b]pyrane, Verfahren zu ihrer Herstellung, ihre Verwendung sowie pharmazeutische Präparate auf Basis dieser Verbindungen

Die vorliegende Erfindung betrifft Benzo[b]pyran-Derivate der Formel I

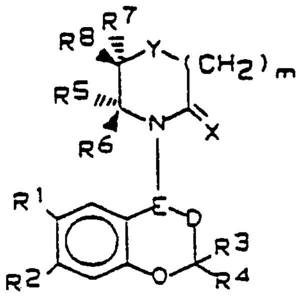

I ,

in welcher
E - D für
a) CH - CH(OH) oder
b) C = CH steht,
X für O oder S,
Y für O, S, SO, $SO_2$ und $NR^9$ steht, wobei $R^9$ für Wasserstoff, ($C_1$-$C_6$)-Alkyl, Phenyl- und Benzyl steht, wobei der Phenylring unsubstituiert oder durch ein oder zwei gleiche oder verschiedene Reste aus der Reihe ($C_1$-$C_2$)-Alkyl, ($C_1$-$C_2$)-Alkoxy, F, Cl, Br, Trifluormethyl, CN, $NO_2$, CO-($C_1$-$C_2$)-Alkyl substituiert ist, bedeutet.
$R^1$ für CN, $NO_2$, F, Cl, Br, $CF_3$, ($C_1$-$C_6$)-Alkoxycarbonyl, $SO_n$-($C_1$-$C_6$)-Alkyl, $SO_n$-Ar, steht; wobei n = 1 oder 2, Ar für ein aromatisches oder heteroaromatisches System steht, das unsubstituiert oder durch 1 bis 3 gleiche oder verschiedene Reste ($C_1$-$C_2$)-Alkyl, ($C_1$-$C_2$)-Alkoxy, F, Cl, Br, Trifluormethyl, CN, $NO_2$, CO-($C_1$-$C_2$)-Alkyl, $SO_p$-($C_1$-$C_2$)-Alkyl, substituiert ist und p für 1 oder 2 steht,
$R^2$ für H, OH, ($C_1$-$C_2$)-Alkoxy, ($C_1$-$C_2$)-Alkyl, F, Cl, Br oder $NR^{10}$ $R^{11}$ steht, wobei $R^{10}$ und $R^{11}$ gleich oder verschieden sind und für H, ($C_1$-$C_5$)-Alkyl oder ($C_1$-$C_5$)-Alkylcarbonyl stehen,
$R^3$ und $R^4$ gleich oder verschieden sind und für Alkyl mit 1-4 C-Atomen stehen,
die oben genannten Bedeutungen von $R^1$ und $R^2$ auch vertauscht sein können,
$R^5$ und $R^6$ gleich oder verschieden sind und für Wasserstoff ($C_1$-$C_8$)-n-Alkyl, ($C_1$-$C_{10}$)iso-Alkyl-, $(CH_2)_{1-6}CO_2$($C_1$-$C_6$)-Alkyl, -$(CH_2)_{1-6}CONR^{10}R^{11}$,
wobei $R^{10}$ und $R^{11}$ in vorstehenden Bedeutungen stehen,
-$(CH_2)_{0-6}CO_2H$, -$(CH_2)_{1-6}$-CO-($C_1$-$C_6$)-Alkyl, -$CO_2$-($C_1$-$C_6$)-Alkyl, ($C_1$-$C_6$)-Alkylmercapto-($C_1$-$C_6$)alkyl-, ($C_1$-$C_6$)-Alkylsulfinyl-($C_1$-$C_6$)alkyl-, ($C_1$-$C_6$)-Alkylsulfonyl-($C_1$-$C_6$)alkyl-, ($C_1$-$C_7$)-Hydroxyalkyl, ($C_1$-$C_7$)-Mercaptoalkyl-, Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,
wobei Ar ein aromatisches oder heteroaromatisches System bedeutet, das unsubstituiert oder durch 1 bis 3 gleiche oder verschiedene Reste aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_p$-($C_1$-$C_2$)Alkyl, substituiert ist,
p für 1 oder 2 steht, und
f null, 1, 2 und 3 bedeutet,
wobei jedoch Verbindungen ausgeschlossen sind, in denen $R^5$ und $R^6$ gleichzeitig Wasserstoff bedeuten,
$R^7$ und $R^8$ gleich oder verschieden sind und für Wasserstoff, ($C_1$-$C_3$)-Alkyl oder Phenyl stehen,
m null, 1 oder 2 bedeutet; ausgeschlossen sind jedoch Verbindungen, in denen gleichzeitig

1. m = 1
2. einer der beiden Substituenten $R^5$ oder $R^6$ gleich Methyl und der andere Wasserstoff, $R^7$ und $R^8$ Wasserstoff und
3. Y Sauerstoff, Schwefel, NH oder N-($C_1$-$C_4$)-Alkyl bedeuten.

Unter einem aromatischen System Ar wird vorzugsweise Phenyl, Naphthyl oder Biphenyl verstanden, ein 5 oder 6-gliedriges heteroaromatisches System Ar ist vorzugsweise ein Rest eines 5 oder 6-gliedrigen O-, N- und/oder S-heterocyclischen Ringes, insbesondere Furyl, Thienyl, Isothiazolyl, Oxazolyl, Isoxazolyl, Pyrazolyl, Imidazolyl, Indolyl, Thiazolyl, Pyridyl, Pyrazinyl, Pyrimidinyl, Pyridazinyl, Triazinyl.

Alkyl und davon abgeleitet Reste wie Alkoxy, Alkylmercapto, Alkylsulfinyl, Alkylsulfonyl können geradkettig oder verzweigt sein.

Unter Halogen wird F, Cl, Br oder J, vorzugsweise F und Cl verstanden.

In Verbindungen der Formel I, in denen E-D für a) CH-CH(OH) steht, sind die C-Atome 3 und 4 des 3,4-Dihydro-2H-benzo[b]pyransystems (nachfolgend auch als "Chromansystem" bezeichnet) asymmetrisch substituiert. Die Erfindung betrifft nur solche Verbindungen, die an diesen Zentren entgegengesetzte Konfigurationen aufweisen. Dies bedeutet, daß der heterocyclische Amid- bzw. Thioamidring als Substituent an C-4 und die OH-Gruppe an C-3 stets "trans" zueinander orientiert sind.

Aus den Definitionen für $R^5$ bis $R^8$ geht hervor, daß der heterocyclische Amid-/Thioamidteil zwei weitere Asymmetriezentren in $\alpha$- und $\beta$-Position zum N-Atom tragen kann.

Die Erfindung betrifft dabei sowohl Verbindungen mit R- als auch S-konfigurierten Zentren. Gleiches gilt für den Fall, daß $R^1$, $R^2$, $R^3$ oder $R^4$ Asymmetriezentren enthalten oder selbst als Substituenten ein Asymmetriezentrum erzeugen. Die Verbindungen können dann als optische Isomere, als Diastereoisomere, als Racemate oder als Gemisch derselben vorliegen.

Mit den erfindungsgemäßen Verbindungen der Formel I wurde eine Substanzklasse mit blutdrucksenkenden Eigenschaften gefunden, die sich von den bekannten vor allem dadurch unterscheidet, daß ein heterocyclischer Amid-bzw. Thioamid-Substituent an C-4 des Chromansystems zusätzliche Substituenten $R^5$ und $R^6$ sowie $R^7$ und $R^8$ in den obengenannten Definition trägt. Diese Substitution führt im Vergleich zu den bereits bekannten Verbindungen bei einigen Verbindungen I zu einer beträchtlichen Steigerung der antihypertensiven Wirkung. Zusätzlich wurde beobachtet, daß in einigen Fällen eine solche Wirksteigerung begleitet wird von einer Verminderung der akuten Toxizität, woraus sich insgesamt eine Verbesserung der therapeutischen Breite ableiten läßt. Dies ist gerade bei einer langfristigen Therapie wie die der Hypertoniebehandlung, wo ein Medikament unter Umständen lebenslang eingenommen werden muß, von überragender Bedeutung. Tierexperimentelle Untersuchungen zeigen, daß die Verbindungen der Formel I sich für die Behandlung des gestörten cardiovaskulären Systems eignen, beispielsweise für die Behandlung der Hypertonie, der Herzinsuffizienz oder von Durchblutungsstörungen des Coronarsystems wie etwa Angina. Cerebrale und periphere Durchblutungsstörungen werden ebenfalls günstig beeinflußt. Darüber hinaus beeinflussen andere Verbindungen I, die schwach ausgeprägte Kreislaufeffekte bewirken, glattmuskuläre Organe wie Uterus, Bronchien, Darm und Galle, die ableitenden Harnwege (Ureter, Harnblase und Urethra) im Sinne eines Spasmolyse.

Sie eignen sich deshalb auch zur Behandlung von Krankheiten, die mit Spasmen dieser Organe verbunden sind, beispielsweise zur Behandlung von vorzeitiger Wehentätigkeit bei der Schwangerschaft, von Koliken der Harnleiter oder der Galle, von obstruktiven Atemwegserkrankungen wie Asthma, von Störungen der Darmmotilität wie etwa des irritablen Colons oder der Inkontinenz der Harnblase.

Bevorzugt sind Verbindungen der Formel I, bei denen $R^1$, $R^2$, $R^3$ und $R^4$ die oben genannten Bedeutungen aufweisen, einer der beiden Substituenten $R^5$ und $R^6$ Wasserstoff oder Methyl bedeutet und der andere in vorstehender Bedeutung steht und $R^7$, $R^8$, $R^9$, $R^{10}$, $R^{11}$, m, f, X und Y die obige Bedeutung aufweisen.

Im besonderen bevorzugt sind Verbindungen der Formel I, in denen

$R^1$ für CN, $NO_2$, $SO_2$-($C_1$-$C_4$)-Alkyl,($C_1$-$C_4$)-Alkoxycarbonyl, Fluor Chlor, $CF_3$,

$R^2$ für H und ($C_1$-$C_2$)-Alkoxy,

$R^3$ und $R^4$ für Methyl und Ethyl,

einer der beiden Substituenten $R^5$ und $R^6$ für Wasserstoff oder Methyl und der andere für ($C_1$-$C_8$)-n-Alkyl, ($C_1$-$C_{10}$)-iso-Alkyl, $-(CH_2)_{1-6}CO_2$($C_1$-$C_6$)-Alkyl, $-(CH_2)_{1-6}CONR^{10}R^{11}$, wobei $R^{10}$ und $R^{11}$ in obigen Bedeutungen stehen,

$-(CH_2)_{1-6}-CO_2H$, $-(CH_2)_{1-6}$-CO-($C_1$-$C_6$)-Alkyl, $-CO_2H$, ($C_1$-$C_6$)-Alkylmercapto-($C_1$-$C_6$)alkyl-, ($C_1$-$C_6$)-Alkylsulfinyl-($C_1$-$C_6$)alkyl-, ($C_1$-$C_6$)-Alkylsulfonyl-($C_1$-$C_6$)alkyl-, ($C_1$-$C_7$)-Hydroxyalkyl, ($C_1$-$C_7$)-Mercaptoalkyl-, Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,

wobei Ar ein aromatisches oder heteroaromatisches System bedeutet, das unsubstituiert oder durch 1 bis 3 gleich oder verschiedene Reste aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_p$-($C_1$-$C_2$)Alkyl, substituiert ist,

p für 1 oder 2 steht, und

f null, 1, 2 und 3 bedeutet,

$R^7$ und $R^8$ gleich oder verschieden sind und für Wasserstoff ($C_1$-$C_3$)-Alkyl und Phenyl stehen,

$R^9$ für H, ($C_1$-$C_4$)-Alkyl, Phenyl und Benzyl steht, wobei der Phenylring unsubstituiert oder durch ein oder zwei gleiche oder verschiedene Reste aus der Reihe ($C_1$-$C_2$)-Alkyl, ($C_1$-$C_2$)-Alkoxy, F, Cl, Trifluormethyl, CN, $NO_2$, CO-($C_1$-$C_2$)-Alkyl substituiert ist

m = null oder 1 bedeutet,
X für O oder S und
Y für O, SO, $SO_2$ oder $NR^9$ steht, worin $R^9$ wie oben definiert ist.

Ebenfalls im besonderen bevorzugt sind Verbindungen der Formel I, in denen
$R^1$ für $SO_2$-Phenyl steht, wobei Phenyl unsubstituiert oder durch 1 bis 3 Substituenten aus der Reihe ($C_1$-$C_2$)-Alkyl, ($C_1$-$C_2$)-Alkoxy, F, Cl, Br, Trifluormethyl, CN, $NO_2$, CO-($C_1$-$C_2$)-Alkyl, $SO_P$-($C_1$-$C_2$)-Alkyl, substituiert ist und p für 1 oder 2 steht,
$R^2$ für H und ($C_1$-$C_2$)-Alkoxy steht,
$R^3$ und $R^4$ für Methyl und Ethyl steht,
einer der beiden Substiuenten $R^5$ und $R^6$ für Wasserstoff oder Methyl und der andere für ($C_1$-$C_8$)-n-Alkyl, ($C_1$-$C_{10}$)-iso-Alkyl,-$(CH_2)_{1-6}CO_2$($C_1$-$C_6$)-Alkyl, -$(CH_2)_{1-6}CONR^{10}R^{11}$, wobei $R^{10}$ und $R^{11}$ in obigen Bedeutungen stehen,
-$(CH_2)_{1-6}$-$CO_2H$, -$(CH_2)_{1-6}$-CO-($C_1$-$C_6$)-Alkyl, -$CO_2H$, ($C_1$-$C_6$)-Alkylmercapto-($C_1$-$C_6$)alkyl-, ($C_1$-$C_6$)-Alkylsulfinyl-($C_1$-$C_6$)alkyl-, ($C_1$-$C_6$)-Alkylsulfonyl-($C_1$-$C_6$)alkyl-, ($C_1$-$C_7$)-Hydroxyalkyl, ($C_1$-$C_7$)-Mercaptoalkyl-, Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,
wobei Ar ein aromatisches oder heteroaromatisches System bedeutet, das unsubstituiert oder durch 1 bis 3 gleiche oder verschiedene Reste aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_p$-($C_1$-$C_2$)Alkyl, substituiert ist,
p für 1 oder 2 steht, und
f null, 1, 2 und 3 bedeutet,
$R^7$ und $R^8$ gleich oder verschieden sind und für Wasserstoff ($C_1$-$C_3$)-Alkyl und Phenyl stehen,
$R^9$ für H, ($C_1$-$C_4$)-Alkyl, Phenyl und Benzyl steht, wobei der Phenylring unsubstituiert oder durch einen oder zwei gleiche oder verschiedene Reste aus der Reihe ($C_1$-$C_2$)-Alkyl, ($C_1$-$C_2$)-Alkoxy, F, Cl, Trifluormethyl, CN, $NO_2$, CO-($C_1$-$C_2$)-Alkyl substituiert ist,
m = null oder 1 bedeutet,
X für O oder S und
Y für O, SO, $SO_2$ oder $NR^9$, worin $R^9$ in der angegebenen Bedeutung steht.

Ganz besonders bevorzugt sind Verbindungen der Formel I, in denen E-D wie oben unter a) definiert ist und
$R^1$ für CN oder $SO_2CH_3$,
$R^2$ für H,
$R^3$ und $R^4$ für Methyl stehen,
einer der Substituenten $R^5$ und $R^6$ Wasserstoff bedeutet und der andere für ($C_1$-$C_6$)-n-Alkyl, ($C_1$-$C_8$)-iso-Alkyl, Phenyl, Benzyl, Pyridyl steht, wobei die aromatischen bzw. heteroaromatischen Systeme unsubstituiert oder mit einem Rest aus der Reihe ($C_1$-$C_2$)-Alkyl, ($C_1$-$C_2$)-Alkoxy, Chlor, Fluor, $CF_3$ und CN substituiert sind, $R^7$ und $R^8$ für Wasserstoff stehen,
m = null bedeutet,
X für O und
Y für O oder $NR^9$ steht, worin $R^9$ ($C_1$-$C_4$)-Alkenyl und Benzyl bedeutet.

Ebenfalls ganz besonders bevorzugt sind Verbindungen der Formel I, in denen E-D wie oben unter a) definiert ist und
$R^1$ für $SO_2$-Phenyl,
$R^2$ für Wasserstoff,
$R^3$ und $R^4$ für Methyl stehen,
einer der Substituenten $R^5$ und $R^6$ Wasserstoff bedeutet und der andere für ($C_1$-$C_6$)-n-Alkyl, ($C_1$-$C_8$)-iso-Alkyl, Phenyl, Benzyl, Pyridyl steht, wobei die aromatischen bzw. heteroaromatischen Systeme unsubstituiert oder mit einem Rest aus der Reihe ($C_1$-$C_2$)-Alkyl, ($C_1$-$C_2$)-Alkoxy, Chlor, Fluor, $CF_3$ und CN, substituiert sind, $R^7$ und $R^8$ für Wasserstoff stehen,
m = null bedeutet
X für O und
Y für O oder $NR^9$ stehen worin $R^9$ ($C_1$-$C_4$)-Alkyl und Benzyl bedeutet.

Die Erfindung betrifft weiterhin ein Verfahren zur Herstellung der Verbindungen I, in denen E-D wie unter a) definiert ist, daß dadurch gekennzeichnet, daß man

a) eine Verbindung der Formel II

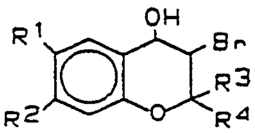

II,

in der $R^1$, $R^2$, $R^3$ und $R^4$ die obengenannten Bedeutungen haben, umsetzt mit einem Lactam bzw. Thiolaktam der Formel III

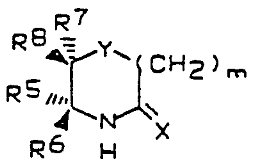

III,

in der $R^5$, $R^6$, $R^7$, $R^8$, X, Y und m die obengenannten Bedeutungen haben, oder daß man eine

b) Verbindung der Formel IV,

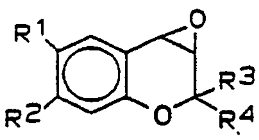

IV

in der $R^1$, $R^2$, $R^3$ und $R^4$ die oben genannten Bedeutungen haben, umsetzt mit einem heterocyclischen Amid bzw. Thioamid der Formel III,

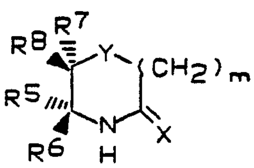

III

oder daß man

c) eine Verbindung der Formel II,

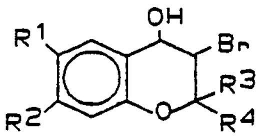

II,

in der $R^1$, $R^2$, $R^3$ und $R^4$ die obengenannten Bedeutungen haben, umsetzt mit einer Verbindung der Formel V

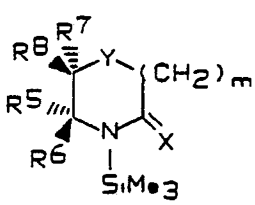

V

in der $R^5$, $R^6$, $R^7$, $R^8$, X, Y und m die obengenannten Bedeutungen haben, oder daß man

d) eine Verbindung der Formel IV

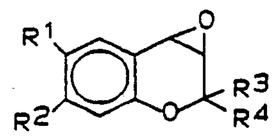

IV

in der $R^1$, $R^2$, $R^3$ und $R^4$ die obigen Bedeutungen aufweisen, mit einer Verbindung der Formel V,

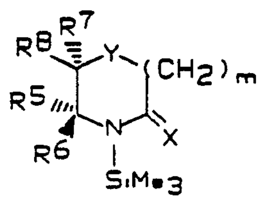

V,

in der $R^5$, $R^6$, $R^7$, $R^8$, X, Y und m die obengenannten Bedeutungen haben, zur Reaktion bringt, oder daß man

e) eine Verbindung der Formel VI,

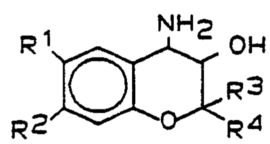

VI

in der $R^1$, $R^2$, $R^3$ und $R^4$ die obengenannten Bedeutungen haben, acyliert zur Verbindung der Formel VII,

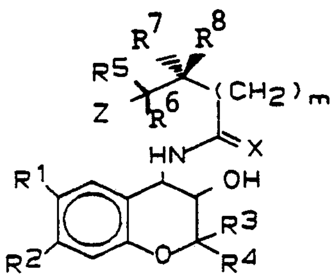

VII

in der $R^1$, $R^2$, $R^3$, $R^4$, $R^5$, $R^6$, $R^7$, $R^8$, X und m wie oben definiert sind und Z die Bedeutung einer Fluchtgruppe wie Chlor oder Brom hat,
und diese cyclisiert zu einer Verbindung der Formel I.

Die unter a)- d) angegebenen Verfahren eignen sich gleichermaßen zur Herstellung von Verbindungen der Formel I, in denen E-D wie unter b) definiert ist.

Werden die Verbindungen I nach den Methoden a) und b) hergestellt, so geschieht dies dadurch, daß man die Verbindungen II und IV in einem geeigneten Lösemittel wie etwa Dimethylsulfoxid, Dimethylformamid, Tetrahydrofuran oder N-alkylierten Harnstoffen, beispielsweise 1-3-Dimethyltetrahydro-2(1H)-pyrimidinon (DMPU), umsetzt mit den Verbindungen III, vorzugsweise unter Einwirkung von starken Basen wie etwa Natriumhydrid, Kaliumhydrid, Lithiumdiisopropylamid, Lithium-bis-(trimethylsilyl)-amid, Kalium-bis-(dimethylsilyl)amid oder ähnlichen für Lactam-N-Alkylierungen bekanntermaßen geeigneten Basen. Die Reaktionstemperatur ist dabei im weiten Bereich variierbar, vorzugsweise wird zwischen 0 °C und Zimmertemperatur gearbeitet oder bei Temperaturen, die leicht oberhalb der Zimmertemperatur liegen können.

Verbindungen, die nach den Methoden a) oder b) nur schwierig herstellbar sind, können nach den Verfahren c) oder d) zugänglich gemacht werden. In diesem Fall rührt man die Verbindungen II oder IV zusammen mit den Verbindungen V in Gegenwart eines Desilylierungsmittels wie Kalium-tert.-butylat oder Tetrabutylammoniumfluorid-trihydrat in einem dipolar aprotischen Lösemittel wie THF und dergleichen. Es ist auch möglich, die Umsetzung auch ohne Lösemittelzusätze in Gegenwart eines Überschusses der teils flüssigen Verbindungen V durchzuführen. Die Temperatur kann dabei in weiten Grenzen variieren.

So erhält man in vielen Fällen bereits bei Zimmertemperatur die erfindungsgemäßen Verbindungen I, in anderen Fällen erst nach Erhitzen auf 60 - 80 °C. In einzelnen Fällen sind sogar noch höhere Temperaturen notwendig.

Verbindungen der Formel III sind in vielen Fällen käuflich oder können einfach nach literaturbekannten Methoden synthetisiert werden.

Die Silylverbindungen der Formel V können in an sich bekannter Weise aus Verbindungen der Formel III hergestellt werden, beispielsweise durch Erhitzen mit 1,1,1,3,3,3-Hexamethyldisilazan und anschließende Destillation.

Bei Verwendung von racemischen oder auch optisch einheitlichen heterocyclischen Amiden- bzw. Thioamiden der Formel III bzw. ihrer Silylderivate der Formel V werden wenigstens 2 neue Produkte der Formel I erhalten. Diese Produkte können durch die üblichen Methoden, wie Kristallisation oder Chromatographie, getrennt werden, in vielen Fällen hat sich auch eine Kombination beider Methoden als günstig erwiesen. Den Produkten kann dann durch gängige physikalische Untersuchungen, wie etwa die Röntgenstrukturanalyse oder die NMR-Spektroskopie, die jeweilige Gesamtkonfiguration zugeordnet werden. Optisch einheitliche, also enantiomerenreine Verbindungen I können durch anschließende Racematspaltung erhalten werden. Werden jedoch bereits enantiomerenreine, heterocyclische Amide bzw. Thioamide III oder die Silylderivate V verwendet, so erhält man die diastereoisomeren Verbindungen I ebenfalls in enantiomerenreiner Form und eine Racematspaltung wird überflüssig.

Die Bromhydrine II bzw. die Epoxide IV sind in vielen Fällen bekannt (vgl. hierzu die unten zitierten Patentschriften oder J. Med. Chem. 1986, 29, 2194-2201) oder können analog nach dort angegebenen Methoden hergestellt werden.

Die heterocyclischen Amide bzw. Thioamide der Formel III sind in vielen Fällen bekannt oder können leicht nach literaturbekannten Methoden hergestellt werden. Beispielsweise können Verbindungen der Formel III, in denen Y = 0, X = 0 und m = null bedeutet, erhalten werden, indem man die 2-Amino-1-alkanole der Formel VIII mit Phosgenäquivalenten, wie Diphenylcarbonat, Diethylcarbonat, N,N-Carbonyldiimidazol oder Chlorameisensäuretrichlormethylester in einem geeigneten Lösungsmittel zur Reaktion bringt (vgl. z. B. J. Org. Chem. 1985, 50, 1830-1835).

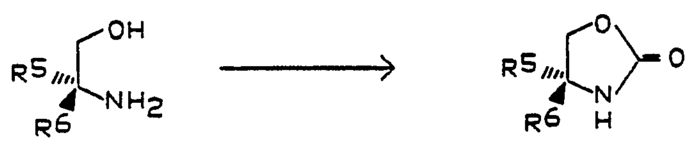

VIII IX

Analoge Verbindungen, in denen X = S bedeutet, werden entsprechend mit Thiophosgenäquivalenten erhalten.

Verbindungen der Formel III, in denen Y = $NR^9$, X = O oder S und m = null bedeutet, können beispielsweise nach folgenden Formelschema hergestellt werden.

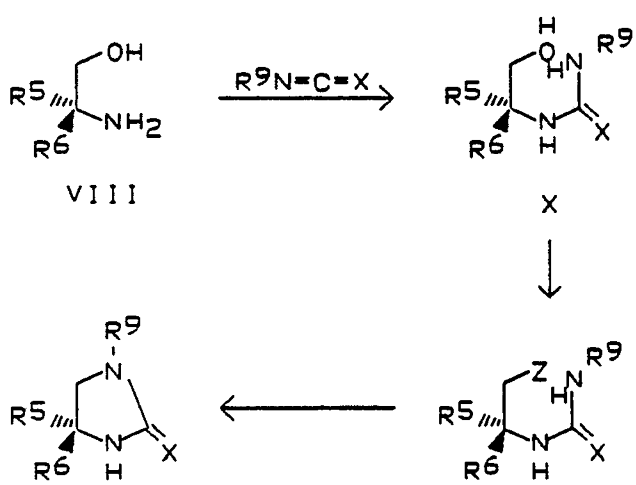


VIII X

III XI

Substituierte 2-Amino-1-alkanole der Formel VIII werden mit Isocyanaten bzw. Isothiocyanaten in die Harn- bzw. Thioharnstoffe der Fomel X überführt, aus denen mit Halogenierungsreagenzien wie Thionylchlorid die Verbindungen XI erhalten werden. Durch anschließende Ringschlußreaktion sind die substituierten Imidazolidin-2-one der Formel III zugänglich.

In J. Med. Chem. 1986, 29, 2194-2201 werden Verbindungen beschrieben, die den erfindungsgemäßen Verbindungen nahe stehen. Sie sind dort unter folgenden allgemeinen Formeln zusammengefaßt:

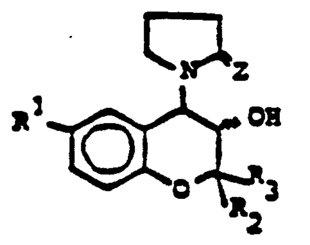

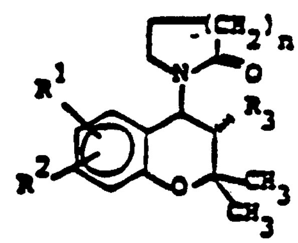

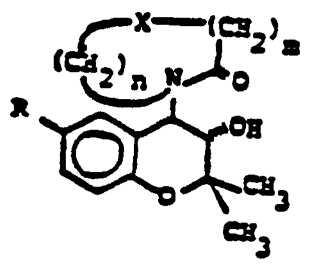

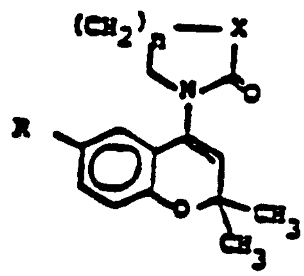

mit $R^1$, $R^2$, $R^3$, X, Z, n, m, R in den dort aufgeführten Bedeutungen. Ein großer Teil dieser Verbindungen ist auch in verschiedenen Patentanmeldungen beschrieben, zu nennen sind hierbei: EP 107 423, EP 120 427, EP 76 075, EP 120 428, EP 277 611.

In EP 107 423 und in EP 277 611 werden Verbindungen beschrieben, die den erfindungsgemäßen Verbindungen am nächsten stehen.

Von diesen Verbindungen ist bekannt, daß sie einen krankhaften erhöhten Blutdruck zu senken vermögen, indem sie die glatte Gefäßmuskulatur relaxieren bzw. gegenüber pressorischen Reizen schützen oder unempfindlich machen können.

In EP 0 107 423 werden Chroman-Derivate der Formel A

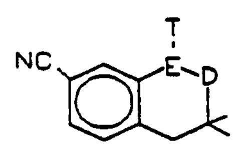

A

beschrieben, wobei
T 3-oxazolidinyl-2-on,3-thiazolidinyl-2-on, 1-morpholinyl-2-on, 1-thiomorpholinyl-2-on und N-substituiertes 1-Piperazinyl-2-on und E-D CH-CH(OH) oder CH=CH bedeuten kann.

Diese Verbindungen tragen jedoch keinen Substituenten im heterocyclischen Amid- bzw. Thioamidring.

In EP 277 611 sind Verbindungen der Formel B

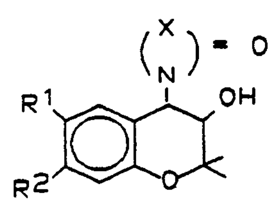

B

beschrieben,

in welcher X für eine Kette $(CH_2)_m$ steht, die durch eine $(C_1-C_2)$-Alkylgruppe substituiert ist sowie durch ein Heteroatom Y, das für O, S oder NR′ steht, unterbrochen sein kann und wobei m für 2, 3 oder 4 steht. Definitionsgemäß werden somit nur Verbindungen beschrieben, in denen ein Heteroatom Y Mittelglied in einer $(CH_2)_m$-Kette ist, nicht aber endständig ist, wie es die vorliegende Erfindung für den Fall, daß m Null bedeutet, ausschließlich beansprucht.

Die hier beschriebenen heterocyclischen Amide und Thioamide bieten den Vorteil, daß zu ihrer Synthese u.a. substituierte, optisch aktive 2-Amino-1-alkanole nach literaturbekannten Reduktionsverfahren aus natürlichen und synthetischen α-Aminosäuren und deren Derivaten wohlfeil zur Verfügung stehen.

Der Vorteil der Verwendung von aus chiralen 2-Amino-1-alkanolen hergestellten, heterocyclischen Amidbausteinen, wie substituierten Imidazolidin-2-onen und 4-substituierten Oxazolidin-2-onen, besteht darin, daß man die diastereoisomeren Verbindungen der Formel I ebenfalls in enantiomerenreiner Form erhält und eine Racematspaltung überflüssig wird.

Die Verwendung der Derivate der enantiomerenreinen α-Aminosäuren ermöglicht nicht nur die gezielte Vorgabe der Konfiguration in α-Position zum Amid-N-Atom, sondern gewährleistet insbesondere die Einführung einer Vielzahl von Substituenten $R^5$ und $R^6$, wobei in diesen Fällen einer der Substituenten Wasserstoff bedeutet.

Einer der Substituenten $R^5$ und $R^6$ läßt sich somit in einer Anzahl von Fällen auf die variablen Reste der α-Aminosäuren zurückzuführen, beispielsweise läßt sich ein Substituentenmuster

| | |
|---|---|
| $R^5$ = Methyl<br>$R^6$ = H | auf<br>D-Alanin, |
| $R^5$ = H<br>$R^6$ = Methyl | auf<br>L-Alanin, |
| $R^5$ = isopropyl<br>$R^6$ = H | auf<br>D-Valin |

und sofort, zurückzuführen.

Die erfindungsgemäßen Verbindungen der Formel I sind, wie bereits erwähnt, Antihypertensiva und Spasmolytika, die als Pharmazeutika in der Human- und Veterinärmedizin eingesetzt werden können. Sie werden in Dosierungen von mindestens 0,001 mg/kg/Tag, vorzugsweise 0,005 mg und insbesondere 0,5 mg/kg/Tag bis maximal 10 mg/kg/Tag, vorzugsweise 5 mg/kg/Tag und insbesondere 2 mg/kg/Tag in Kapseln, Dragees, Tabletten, Pudern, Zäpfchen oder Lösungen mit Zusätzen oder ohne Zusätze von galenischen Hilfsstoffen enteral, z.B. oral oder parenteral (wie etwa durch Injektion in das Gefäßsystem, z.B. intravenös) verabfolgt, jeweils bezogen auf ein Gewicht von ca. 75 kg. Sie eignen sich zur Behandlung der Hypertonie, allein oder in Kombination mit anderen antihypertensiv wirkenden Arzneimitteln, wie etwa Diuretika, Ca-Antagonisten, ACE-Hemmern oder ß-Blockern. Diese Angaben beziehen sich auf einen Menschen vom Gewicht von 75 kg.

Beispiel 1

a) N-Trimethylsilyl-4S-benzyl-oxazolidin-2-on

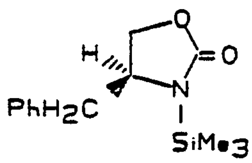

9,9 g (55 mMol) 4S-Benzyloxazolidin-2-on werden unter Argon in 50 ml Hexamethyldisilazan suspendiert und langsam auf 125 °C erhitzt, wobei das anfangs 2-phasige System nach ca. 2,5 h in eine klare Lösung übergeht. Nach weiteren 7,5 h bei 125 °C wird im Vakuum vom überschüssigen Hexamethyldisilazan befreit und der Rückstand an der Ölpumpe destilliert.
$Kp._{0,1}$ = 151 °C,
Farblose Kristalle, Fp. 52 -54 °C.

b) 2,2-Dimethyl-3,4-dihydro-6-phenylsulfonyl-trans[3-hydroxy-4-(4'S-benzyl-3-oxazolidinyl-2-on)]-2H-benzo-[b]pyran Isomer A und B

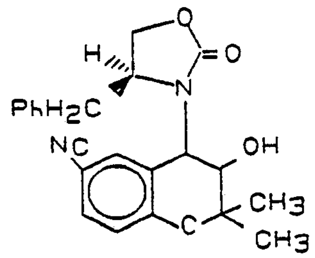

Bei Zimmertemperatur werden unter Rühren portionsweise 31,5 g (20 mMol) Tetra-n-butylammoniumfluorid-Trihydrat zu einer Lösung von 4,0 g (20 mMol) 2,2-Dimethyl-3,4-dihydro-3,4-epoxy-6-cyano-2H-benzo[b]pyran und 5,5 g (22 mMol) N-Trimethylsilyl-4S-benzyloxazolidin-2-on in 100 ml N,N-Dimethylacetamid hinzugeben. Anschließend wird 2 Stunden auf 70 °C erwärmt, bei Zimmertemperatur in 200 ml wäßrige $NH_4Cl$-Lösung eingetragen. Nach Extrahieren mit Ethylacetat, Trocknen und Einengen im Vakuum wird der Rückstand mit Cyclohexan, Ethylacetat (1:1) an Kieselgel chromatographiert. Entsprechende Fraktionen werden eingeengt und das Produktgemisch der beiden Isomeren mit Ethylacetat zur Kristallisation gebracht. Laut $^1$H-NMR (270 MHz) liegt nahezu ein 50 : 50-Gemisch von Isomer A und B vor. Fp. 188 - 190 °C

Beispiel 2

a) 4R-Ethyl-oxazolidin-2-on

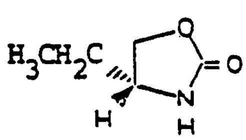

Zu 14,8 g (0,15 Mol) R-(-)-2-Amino-1-butanol in 200 ml wasserfreiem Dioxan gibt man unter Rühren bei Zimmertemperatur portionsweise 24,3 g (0,15 Mol) N,N-Carbonyldiimidazol und erwärmt 1 h auf 80 °C, dann 1 h zum Rückfluß. Nach Einengen im Vakuum wird auskristallisiertes Imidazol abgesaugt, das Filtrat mit 100 ml 1N HCl versetzt, dreimal mit Dichlormethan extrahiert und die organische Phase über $MgSO_4$ getrocknet. Nach Abdestillieren des Lösungmittels erhält man ein öliges Produkt.
$^1$H-NMR ($CDCl_3$, 60 MHz):
$\delta$ = 6,70 (s (br) NH), 4,23 (t, 1H), 3,80 (m,2H) 1,55 (m, $CH_2$), 0,93 (t, $CH_3$).

b) 2,2-Dimethyl-4-(4'R-ethyl-3-oxazolidinyl-2-on)-6-phenyl-sulfonyl-2H-benzo[b]pyran

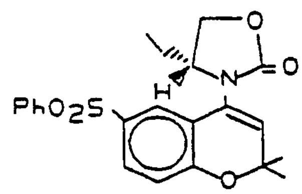

Unter Stickstoffatmosphäre werden zu 4,6 g (40 mMol) 4R-Ethyl-oxazolidin-2-on in 70 ml N,N-Dimethylacetamid unter Rühren portionsweise 4,5 g (40 mMol) Kalium-tert.butylat zugegeben. Nach 30 Minuten werden bei Zimmertemperatur 11,0 g (33 mMol) 2,2-Dimethyl-3,4-dihydro-3,4-epoxy-6-phenylsulfonyl-2H-benzo[b]pyran gelöst in 25 ml N,N-Dimethylacetamid zugetropft. Anschließend erwärmt man 1,5 Stunden auf 65 °C, läßt abkühlen und trägt in 2 l Eiswasser ein. Das auskristallisierte Rohprodukt wird abgesaugt, in Dichlormethan aufgenommen, getrocknet und mit Cyclohexan /Ethylacetat(1:1) an Kieselgel chromatographiert. Entsprechende Fraktionen werden eingeengt und das Produkt mit Diethylether zur Kristallisation gebracht.
Fp. 149 - 151 °C

Beispiel 3

2,2-Dimethyl-3,4-dihydro-trans[3-hydroxy-4-(4'R-ethyl-3-oxazolidinyl-2-on)]-6-phenylsulfonyl-2H-benzo[b]-pyran, Isomer A

2,2-Dimethyl-3,4-dihydro-trans[3-hydroxy-4-(4'R-ethyl-3-oxazolidinyl-2-on)]-6-phenylsulfonyl-2H-[b]pyran, Isomer B

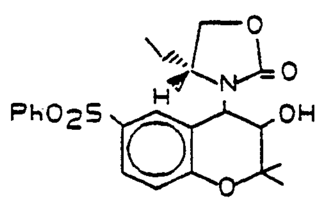

Analog Beispiel 1b) werden 6,3 g (20 mMol) 2,2-Dimethyl-3,4-dihydro-3,4-epoxi-6-phenylsulfonyl-2H-benzo[b]pyran, 4,1 g (22m Mol) N-Trimethylsilyl-4R-ethyl-oxazolidinyl-2-on (analog Beispiel 1a) hergestellt) und 6,9 g (22 mMol) Tetra-n-butylammoniumfluorid-Trihydrat umgesetzt, wobei das Reaktionsgemisch 3 h bei 90 °C gerührt wird. Nach chromatographischer Trennung des Rohproduktes mit Cyclohexan/Ethylacetat (2:1) an Kieselgel (30-70 µm) wird aus entsprechenden Fraktionen Isomer A erhalten, das mit Diethylether zur Kristallisation gebracht wird, Fp. 175 - 177 °C
$^1$H-NMR ($CDCl_3$, 270 MHz): $\delta$ = 6,88 (d, J=9Hz, 1H), 1,55 (s, C-CH$_3$, 1,23 (s,C-CH$_3$), 0,67 (t, -CH$_2$CH$_3$)
Aus entsprechenden Fraktionen wird Isomer B zur Kristallisation gebracht.
Fp. 153 - 155 °C (Diethylether)
$^1$H-NMR ($CDCl_3$, 270 MHz): $\delta$ = 6,93 (d, J=9Hz, 1H), 1,48 (s, C-CH$_3$), 1,23 (s, C-CH$_3$), 0,84 (t, -CH$_2$CH$_3$).

Beispiel 4

2,2-Dimethyl-3,4-dihydro-trans[3-hydroxy-4-(4'R-ethyl-3-oxazolidinyl-2-on)]-6-cyano-2H-benzo[b]pyran, Isomer A

2,2-Dimethyl-3,4-dihydro-trans[3-hydroxy-4-(4'R-ethyl-3-oxazolidinyl-2-on)]-6-cyano-2H-benzo[b]pyran, Iso-

mer B

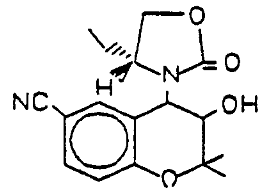

Analog Beispiel 1b) werden 5,0 g (25 mMol) 2,2-Dimethyl-3,4-dihydro-3,4-epoxi-6-cyano-2H-benzo[b]-pyran, 4,8 g (25 mMol) N-Trimethylsilyl-4R-ethyl-oxazolidinyl-2-on und 8 g (25 mMol) Tetra-n-butylammoniumfluorid-Trihydrat umgesetzt, wobei das Reaktionsgemisch 1,5 Stunden bei 60 °C und 2 h bei 90 °C gerührt wird, N,N-Dimethylacetamid wird weitgehend im Vakuum abdestilliert, der Rückstand mit gesättigter wäßriger $NH_4Cl$-Lösung versetzt und mit Ethylacetat extrahiert. Nach chromatographischer Trennung des Rohprodukts mit Toluol/Ethylacetat am Kieselgel (30-70 µm) wird aus entsprechenden Fraktionen Isomer A und Isomer B erhalten, die mit Diethylether zur Kristallisation gebracht werden. **Isomer A:**

Fp. 208 - 210 °C

$^1H$-NMR ($CDCl_3$, 270 Hz): δ = 7,53 (s, 1H), 7,44 (m, 1H), 6,87 (d, J=9Hz, 1H), 1,58 (s, $CH_3$), 1,27 (s, $CH_3$), 0,77 (t, $CH_2C\underline{H}_3$).

**Isomer B:**

Fp. 166 - 168 °C (Diethylether)

$^1$ H-NMR ($CDCl_3$, 270 Hz): δ = 7,46 (m, 1H), 7,40 (s, 1H), 6,90 (d, J=9Hz, 1H), 1,58 (s, $CCH_3$), 1,27 (s, $CH_3$), 0,94 (t, $CH_2C\underline{H}_3$).

Beispiel 5

2,2-Dimethyl-4-(4'R-ethyl-3-oxazolidinyl-2-on)-6-cyano-2H-benzo[b]pyran

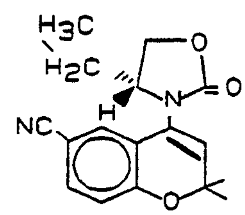

Die Titelverbindung wird wie unter 2b) aus 2,2-Dimethyl-3,4-dihydro-3,4-epoxi-6-cyano-2H-benzo[b]-pyran und 4R-Ethyl-oxazolidin-2-on erhalten; farblose Kristalle Fp. 188 - 189 °C (aus Diethylether)

Beispiel 6

a) 4R-Methyloxazolidin-2-on

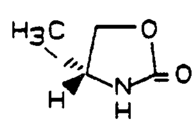

Analog Beispiel 2a) werden 15 g (0,2 Mol) D-Alaninol mit 32,5 g (0,2 Mol) N,N-Carbonyldiimidazol zur Reaktion gebracht,
Fp. 51 - 53 °C

b) 2,2-Dimethyl-4-(4'R-methyl-3-oxazolidinyl-2-on)-6-cyano-2H-benzo[b]pyran

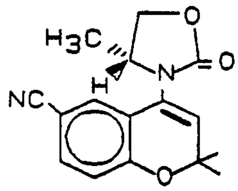

Die Titelverbindung wird wie unter 2b) aus 2,2-Dimethyl-3,4-dihydro-3,4-epoxi-6-cyano-2H-benzo[b]-pyran und 4R-Methyloxazolidin-2-on erhalten; farblose Kristalle Fp. 195 - 197 °C (aus Diethylether)

Beispiel 7

a) N-Trimethylsilyl-4R-methyloxazolidin-2-on

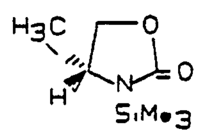

Analog Beispiel 1a) wird die Titelverbindung aus 4R-Methyloxazolidin-2-on und Hexamethyldisilazan erhalten,
$Kp_2$ = 85 °C
Fp. = 46 - 48 °C

b) 2,2-Dimethyl-3,4-dihydro-6-cyano-trans[3-hydroxy-4-(4'R-methyl-3-oxazolidinyl-2-on)]-2H-benzo[b]pyran, Isomer A

2,2-Dimethyl-3,4-dihydro-6-cyano-trans[3-hydroxy-4-(4'R-methyl-3-oxazolidinyl-2-on)]-2H-benzo[b]pyran, Isomer B

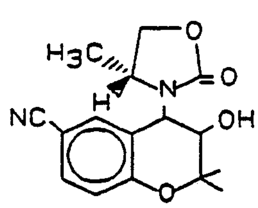

Analog den Beispielen 1 und 4 werden 5 g (25 mMol) 2,2-Dimethyl-3,5-dihydro-3,4-epoxy-6-cyano-2H-benzo[b]pyran und 5,2 g (30 mMol) N-Trimethylsilyl-4R-methyloxazolidin-2-on umgesetzt und das Rohprodukt mit Toluol/Ethylacetat (1:1) chromatographisch an Kieselgel gereinigt.

Aus entsprechenden Fraktionen wird das langsamer laufende Isomer A erhalten,
Fp. 242 - 243 °C (Ethylacetat)

$^1H$-NMR ($CDCl_3$, 270 MHz): $CH_3$ Signale: $\delta$ = 1,58 (s), 1,27 (s), 0,96 (d).

Eine chromatographisch erhaltene Mischfraktion von Isomer A: Isomer B (ca. 1:2) wird in Ethanol in Lösung gebracht und anschließend mit einer gleichen Menge Wasser versetzt. Aus dieser Lösung kristallisiert ein Gemisch aus, in dem der Anteil von A überwiegt. Aus der Mutterlauge wird Isomer B in Form farbloser Kristalle erhalten,

Fp. 203 - 204 °C

$^1H$-NMR ($CDCl_3$, 270 MHz) $CH_3$-Signale $\delta$ = 1,50 (s), 1,35 (d), 1,25 (s).

Beispiel 8

a) N-Trimethylsilyl-4S-methyl-oxazolidin-2-on

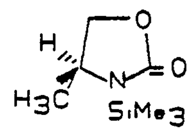

Analog Beispiel 1a) wird die Titelverbindung aus 4S-Methyloxazolidin-2-on erhalten

$Kp_2$ = 84 - 86 °C

Fp. = 46 - 48 °C.

b)2,2-Dimethyl-3,4-dihydro-6-cyano-trans[3-hydroxy-4-(4′S-methyl-3-oxazolidinyl-2-on)]-2H-benzo[b]pyran, Isomer A

2,2-Dimethyl-3,4-dihydro-6-cyano-trans[3-hydroxy-4-(4′S-methyl-3-oxazolidinyl-2-on)]-2H-benzo[b]pyran, Isomer B

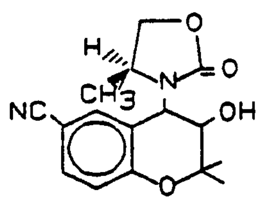

Die Herstellung erfolgt analog den Beispiel 1,4 und 7.

Die Isolierung erfolgt wie in Beispiel 7b) beschrieben durch Säulenchromatographie und Kristallisation

Isomer A:

Fp. 241 - 243 °C

$^1H$-NMR ($CDCl_3$, 270 MHz):$CH_3$-Signale: $\delta$ = 1,58 (s), 1,27 (s), 0,96 (d).

Isomer B:

Fp. 202 - 204 °C

$^1H$-NMR ($CDCl_3$, 270 MHz): $CH_3$-Signale: $\delta$ = 1,50 (s), 1,35 (d), 1,25 (s).

Beispiel 9

2,2-Dimethyl-3,4-dihydro-6-cyano-trans[3-hydroxy-4-(4′R-isopropyl-3-oxazolidinyl-2-on)]-2H-benzo[b]-pyran, Isomer A

Fp. 226-228 °C (aus Ethylacetat)

2,2-Dimethyl-3,4-dihydro-6-cyano-trans[3-hydroxy-4′R-isopropyl-3-oxazolidinyl-2-on)]-2H-benzo[b]pyran, Isomer B
Fp. 173-175 °C (aus Diethylether)

Beispiel 10

2,2-Dimethyl-3,4-dihydro-6-cyano-trans[3-hydroxy-4-(4'S-isopropyl-3-oxazolidinyl-2-on)]-2H-benzo[b]-pyran, Isomer A
Fp. 227-228 °C (aus Diethylether)
2,2-Dimethyl-3,4-dihydro-6-cyano-trans[3-hydroxy-4-(4'S-isopropyl-3-oxazolidinyl-2-on)]-2H-benzo[b]pyran, Isomer B
Fp. 174-175 °C (aus Diethylether)

In analoger Weise lassen sich die folgenden Verbindungen der Formel I, in der E-D wie unter a) definiert für CH-CH(OH) steht, m null, $R^2$, $R^7$ und $R^8$ Wasserstoff, X Sauerstoff und $R^3$ und $R^4$ Methyl bedeuten, herstellen

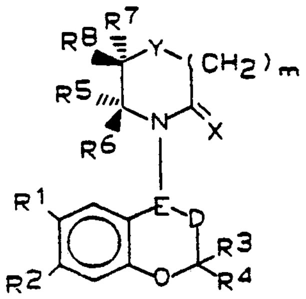

I ,

| $R^1$ | $R^5$ | $R^6$ | Y | Isomer |
|---|---|---|---|---|
| CN | i Prop. | H | NMe | A |
| CN | i Prop. | H | NMe | B |
| $SO_2Me$ | i Prop. | H | O | A |
| $SO_2Me$ | i Prop. | H | O | B |
| $SO_2Ph$ | i Prop. | H | O | A |
| $SO_2Ph$ | i Prop | H | O | B |
| CN | H | i Prop. | NMe | A |
| CN | H | i Prop. | NMe | B |
| $SO_2Me$ | H | i Prop. | O | A |
| $SO_2Me$ | H | i Prop. | O | B |
| $SO_2Ph$ | H | i Prop. | O | A |
| $SO_2Ph$ | H | i Prop. | O | B |
| CN | $CH_2CH(CH_3)_2$ | H | O | A |
| CN | $CH_2CH(CH_3)_2$ | H | O | B |
| $SO_2Ph$ | $CH_2CH(CH_3)_2$ | H | O | A |
| $SO_2Ph$ | $CH_2CH(CH_3)_2$ | H | O | B |
| CN | H | $CH_2CH(CH_3)_2$ | O | A |
| CN | H | $CH_2CH(CH_3)_2$ | O | B |
| $SO_2Me$ | H | $CH_2CH(CH_3)_2$ | O | A |
| $SO_2Me$ | H | $CH_2CH(CH_3)_2$ | O | B |
| CN | H | Et | O | A |
| CN | H | Et | O | B |
| $SO_2Ph$ | H | Et | O | A |
| $SO_2Ph$ | H | Et | O | B |
| $SO_2Me$ | Et | H | O | A |
| $SO_2Me$ | Et | H | O | B |
| CN | Ph | H | O | A |
| CN | Ph | H | O | B |
| $SO_2Me$ | Ph | H | O | A |
| $SO_2Me$ | Ph | H | O | B |
| $SO_2Ph$ | Ph | H | O | A |
| $SO_2Ph$ | Ph | H | O | B |
| CN | H | Ph | O | A |
| CN | H | Ph | O | B |

| $R^1$ | $R^5$ | $R^6$ | Y | Isomer |
|---|---|---|---|---|
| $SO_2Me$ | H | Ph | O | A |
| $SO_2Me$ | H | Ph | O | B |
| $SO_2Ph$ | H | Ph | O | A |
| $SO_2Ph$ | H | Ph | O | B |
| CN | Bz | H | O | A |
| CN | Bz | H | O | B |
| $SO_2Me$ | Bz | H | O | A |
| $SO_2Me$ | Bz | H | O | B |
| $SO_2Ph$ | Bz | H | O | A |
| $SO_2Ph$ | Bz | H | O | B |
| $SO_2Ph$ | H | Bz | O | A |
| $SO_2Ph$ | H | Bz | O | B |
| CN | Me | H | NBz | A |
| CN | Me | H | NBz | B |
| $SO_2Me$ | Me | H | O | A |
| $SO_2Me$ | Me | H | O | B |
| $SO_2Ph$ | Me | H | O | A |
| $SO_2Ph$ | Me | H | O | B |
| CN | H | Me | NBz | A |
| CN | H | Me | NBz | B |
| $SO_2Ph$ | H | Me | O | A |
| $SO_2Ph$ | H | Me | O | B |
| $SO_2Me$ | H | Me | O | A |
| $SO_2Me$ | H | Me | O | B |
| CN | Me | H | NMe | A |
| CN | Me | H | NMe | B |
| CN | H | Me | NMe | A |
| CN | H | Me | NMe | B |
| $SO_2Ph$ | Me | H | N-nBu | A |
| $SO_2Ph$ | Me | H | N-nBu | B |
| CN | Me | H | N-nBu | A |
| CN | Me | H | N-nBu | B |
| CN | i Prop. | H | N-nBu | A |
| CN | i Prop. | H | N-nBu | B |
| CN | Ph | H | NMe | A |
| CN | Ph | H | NMe | B |

**Ansprüche**

1. Benzo[b]pyran-Derivate der Formel I

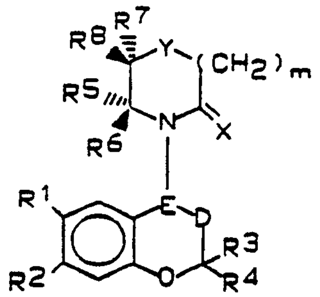

I ,

in welcher
E - D für
a) CH - CH(OH) oder
b) C = CH steht,
X für O oder S,
Y O, S, SO, $SO_2$ und $NR^9$ steht, wobei $R^9$ für Wasserstoff, ($C_1$-$C_6$)-Alkyl, Phenyl- und Benzyl steht, wobei der Phenylring unsubstituiert oder durch ein oder zwei gleiche oder verschiedene Reste aus der Reihe ($C_1$-$C_2$)-Alkyl, ($C_1$-$C_2$)-Alkoxy, F, Cl, Br, Trifluormethyl, CN, $NO_2$, CO-($C_1$-$C_2$)-Alkyl substituiert ist, bedeutet.
$R^1$ für CN, $NO_2$, F, Cl, Br, $CF_3$, ($C_1$-$C_6$)-Alkoxycarbonyl, $SO_n$-($C_1$-$C_6$)-Alkyl, $SO_n$-Ar, steht; wobei n = 1 oder 2, Ar für ein aromatisches oder heteroaromatisches System steht, das unsubstituiert oder durch 1 bis 3 gleiche oder verschiedene Reste ($C_1$-$C_2$)-Alkyl, ($C_1$-$C_2$)-Alkoxy, F, Cl, Br, Trifluormethyl, CN, $NO_2$, CO-($C_1$-$C_2$)-Alkyl, $SO_p$-($C_1$-$C_2$)-Alkyl, substituiert ist und p für 1 oder 2 steht,
$R^2$ für H, OH, ($C_1$-$C_2$)-Alkoxy, ($C_1$-$C_2$)-Alkyl, F, Cl, Br oder $NR^{10}$ $R^{11}$ steht, wobei $R^{10}$ und $R^{11}$ gleich oder verschieden sind und für H, ($C_1$-$C_5$)-Alkyl oder ($C_1$-$C_5$)-Alkylcarbonyl stehen,
$R^3$ und $R^4$ gleich oder verschieden sind und für Alkyl mit 1-4 C-Atomen stehen,
die oben genannten Bedeutungen von $R^1$ und $R^2$ auch vertauscht sein können,
$R^5$ und $R^6$ gleich oder verschieden sind und für Wasserstoff ($C_1$-$C_8$)-n-Alkyl, ($C_1$-$C_{10}$)iso-Alkyl-, $(CH_2)_{1-6}CO_2$($C_1$-$C_6$)-Alkyl, -$(CH_2)_{1-6}CONR^{10}R^{11}$,
wobei $R^{10}$ und $R^{11}$ in vorstehenden Bedeutungen stehen,
-$(CH_2)_{0-6}CO_2H$, -$(CH_2)_{1-6}$-CO-($C_1$-$C_6$)-Alkyl, -$CO_2$-($C_1$-$C_6$)-Alkyl, ($C_1$-$C_6$)-Alkylmercapto-($C_1$-$C_6$)alkyl-, ($C_1$-$C_6$)-Alkylsulfinyl-($C_1$-$C_6$)-alkyl-, ($C_1$-$C_6$)-Alkylsulfonyl-($C_1$-$C_6$)alkyl-, ($C_1$-$C_7$)-Hydroxyalkyl, ($C_1$-$C_7$)-Mercaptoalkyl-, Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,
wobei Ar ein aromatisches oder heteroaromatisches System bedeutet, das unsubstituiert oder durch 1 bis 3 gleiche oder verschiedene Reste aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_p$-($C_1$-$C_2$)Alkyl, substituiert ist,
p für 1 oder 2 steht, und
f null, 1, 2 und 3 bedeutet,
wobei jedoch Verbindungen ausgeschlossen sind, in denen $R^5$ und $R^6$ gleichzeitg Wasserstoff bedeuten,
$R^7$ und $R^8$ gleich oder verschieden sind und für Wasserstoff, ($C_1$-$C_3$)-Alkyl oder Phenyl stehen,
m null, 1 oder 2 bedeutet; ausgeschlossen sind jedoch Verbindungen, in denen gleichzeitig

1. m = 1

2. einer der beiden Substituenten $R^5$ oder $R^6$ gleich Methyl und der andere Wasserstoff, $R^7$ und $R^8$ Wasserstoff und

3. Y Sauerstoff, Schwefel, NH oder N-($C_1$-$C_4$)-Alkyl bedeuten .

2. Verbindungen I nach Anspruch 1, dadurch gekennzeichnet, daß die folgenden Substituenten und Indices folgende Bedeutungen haben:
$R^1$, $R^2$, $R^3$, $R^4$ wie in Anspruch 1 definiert,einer der beiden Substituenten $R^5$ oder $R^6$ ist Wasserstoff oder Methyl und der andere wie in Anspruch 1 definiert,

$R^7$, $R^8$, $R^9$, $R^{10}$, $R^{11}$, m, f, X und Y wie in Anspruch definiert.

3. Verbindungen I nach Anspruch 1, in der
$R^1$ für CN, $NO_2$, $SO_2$-($C_1$-$C_4$)-Alkyl, ($C_1$-$C_4$)-Alkoxycarbonyl, Fluor, Chlor, $CF_3$,
$R^2$ für H und ($C_1$-$C_2$)-Alkoxy,
$R^3$ und $R^4$ für Methyl und Ethyl,
einer der beiden Substiuenten $R^5$ und $R^6$ für Wasserstoff oder Methyl und der andere für ($C_1$-$C_8$)-n-Alkyl, ($C_1$-$C_{10}$)-iso-Alkyl, $(CH_2)_{1-6}CO_2(C_1$-$C_6)$-Alkyl, $-(CH_2)_{1-6}CONR^{10}R^{11}$, wobei $R^{10}$ und $R^{11}$ in obigen Bedeutungen stehen,
$-(CH_2)_{1-6}$-$CO_2H$, $-(CH_2)_{1-6}$-CO-($C_1$-$C_6$)-Alkyl, $-CO_2H$, ($C_1$-$C_6$)-Alkylmercapto-($C_1$-$C_6$)alkyl-, ($C_1$-$C_6$)-Alkylsulfinyl-($C_1$-$C_6$)alkyl-, ($C_1$-$C_6$)-Alkylsulfonyl-($C_1$-$C_6$)alkyl-,($C_1$-$C_7$)-Hydroxyalkyl, ($C_1$-$C_7$)-Mercaptoalkyl-, Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,
wobei Ar ein aromatisches oder heteroaromatisches System bedeutet, das unsubstituiert oder durch 1 bis 3 gleiche oder verschieden Reste aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_P$-($C_1$-$C_2$)Alkyl, substituiert ist,
p für 1 oder 2 steht, und
f null, 1, 2 und 3 bedeutet,
$R^7$ und $R^8$ gleich oder verschieden sind und für Wasserstoff ($C_1$-$C_3$)-Alkyl und Phenyl stehen,
$R^9$ für H, ($C_1$-$C_4$)-Alkyl, Phenyl und Benzyl steht, wobei der Phenylring unsubstituiert oder durch ein oder zwei gleiche oder verschiedene Reste aus der Reihe ($C_1$-$C_2$)-Alkyl, ($C_1$-$C_2$)-Alkoxy, F, Cl, Trifluormethyl, CN, $NO_2$, CO-($C_1$-$C_2$)-Alkyl substituiert ist
m = null oder 1 bedeutet,
X für O oder S und
Y für O, SO, $SO_2$ oder $NR^9$ steht, worin $R^9$ wie oben definiert ist.

4. Verbindung I nach Anspruch 1, dadurch gekennzeichnet, daß
$R^1$ für $SO_2$-Phenyl steht, wobei Phenyl unsubstituiert oder durch 1 bis 3 Substituenten aus der Reihe ($C_1$-$C_2$)-Alkyl, ($C_1$-$C_2$)-Alkoxy, F, Cl, Br, Trifluormethyl, CN, $NO_2$, CO-($C_1$-$C_2$)-Alkyl, $SO_p$-($C_1$-$C_2$)-Alkyl, substituiert ist und p für 1 oder 2 steht,
$R^2$ für H und ($C_1$-$C_2$)-Alkoxy steht,
$R^3$ und $R^4$ für Methyl und Ethyl steht,
einer der beiden Substiuenten $R^5$ und $R^6$ für Wasserstoff oder Methyl und der andere für ($C_1$-$C_8$)-n-Alkyl, ($C_1$-$C_{10}$)-iso-Alkyl,-$(CH_2)_{1-6}$ $CO_2(C_1$-$C_6)$-Alkyl, $-(CH_2)_{1-6}CONR^{10}R^{11}$, wobei $R^{10}$ und $R^{11}$ in obigen Bedeutungen stehen,
$-(CH_2)_{1-6}$-$CO_2H$, $-(CH_2)_{1-6}$-CO-($C_1$-$C_6$)-Alkyl, $-CO_2H$, ($C_1$-$C_6$)-Alkylmercapto-($C_1$-$C_6$)alkyl-, ($C_1$-$C_6$)-Alkylsulfinyl-($C_1$-$C_6$)alkyl-, ($C_1$-$C_6$)-Alkylsulfonyl- ($C_1$-$C_6$)alkyl-, ($C_1$-$C_7$)-Hydroxyalkyl, ($C_1$-$C_7$)-Mercaptoalkyl-, Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,
wobei Ar ein aromatisches oder heteroaromatisches System bedeutet, das unsubstituiert oder durch 1 bis 3 gleiche oder verschiedene Reste aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_P$-($C_1$-$C_2$)Alkyl, substituiert ist,
p für 1 oder 2 steht, und
f null, 1, 2 und 3 bedeutet,
$R^7$ und $R^8$ gleich oder verschieden sind und für Wasserstoff ($C_1$-$C_3$)-Alkyl und Phenyl stehen,
$R^9$ für H, ($C_1$-$C_4$)-Alkyl, Phenyl und Benzyl steht, wobei der Phenylring unsubstituiert oder durch einen oder zwei gleiche oder verschiedene Reste aus der Reihe ($C_1$-$C_2$)-Alkyl, ($C_1$-$C_2$)-Alkoxy, F, Cl, Trifluormethyl, CN, $NO_2$, CO-($C_1$-$C_2$)-Alkyl substituiert ist,
m = null oder 1 bedeutet,
X für O oder S und
Y für O, SO, $SO_2$ oder $NR^9$ mit $R^9$ in der oben angegebenen Bedeutung steht.

5. Verbindung I nach Anspruch 1, dadurch gekennzeichnet, daß
E-D wie oben unter a) definiert ist und
$R^1$ für CN oder $SO_2CH_3$,
$R^2$ für H,
$R^3$ und $R^4$ für Methyl steht,
einer der Substituenten $R^5$ und $R^6$ Wasserstoff bedeutet und der andere für ($C_1$-$C_6$)-n-Alkyl, ($C_1$-$C_8$)-iso-Alkyl, Phenyl, Benzyl, Pyridyl steht, wobei die aromatischen bzw. heteroaromatischen Systeme unsubstituiert oder mit einem Rest aus der Reihe ($C_1$-$C_2$)-Alkyl, ($C_1$-$C_2$)-Alkoxy, Chlor, Fluor, $CF_3$ und CN substituiert sind, $R^7$ und $R^8$ für Wasserstoff steht,

m = null bedeutet,
X für O und
Y für O oder $NR^9$ steht, worin $R^9$ ($C_1$-$C_4$)-Alkyl und Benzyl bedeutet.

6. Verbindung I nach Anspruch 1, dadurch gekennzeichnet, daß
E-D wie oben unter a) definiert ist und
$R^1$ für $SO_2$-Phenyl,
$R^2$ für Wasserstoff,
$R^3$ und $R^4$ für Methyl steht,
einer der Substituenten $R^5$ und $R^6$ Wasserstoff bedeutet und der andere für ($C_1$-$C_6$)-n-Alkyl, ($C_1$-$C_8$)-iso-Alkyl, Phenyl, Benzyl, Pyridyl steht, wobei die aromatischen bzw. heteroaromatischen Systeme unsubstituiert oder mit einem Rest aus der Reihe ($C_1$-$C_2$)-Alkyl, ($C_1$-$C_2$)-Alkoxy, Chlor, Fluor, $CF_3$ und CN, substituiert sind, $R^7$ und $R^8$ für Wasserstoff steht,
m = null bedeutet
X für O und
Y für O oder $NR^9$ steht, worin $R^9$ ($C_1$-$C_4$)-Alk yl und Benzyl bedeutet.

7. Verfahren zum Herstellen einer Verbindung I nach Anspruch 1, dadurch gekennzeichnet, daß man
a) eine Verbindung der Formel II

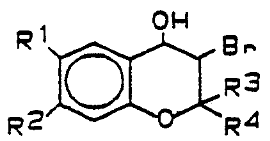

II,

in der $R^1$, $R^2$, $R^3$ und $R^4$ die in Anspruch 1 Bedeutungen haben,
umsetzt mit einem Lactam bzw. Thiolaktam der Formel III

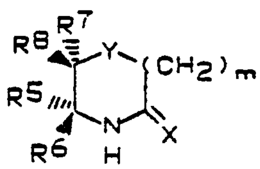

III,

in der $R^5$, $R^6$, $R^7$, $R^8$, X, Y und m die obengenannten Bedeutungen haben, oder daß man eine
b) Verbindung der Formel IV,

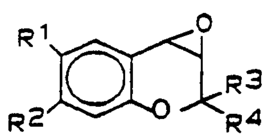

IV

in der $R^1$, $R^2$, $R^3$ und $R^4$ die oben genannten Bedeutungen haben, umsetzt mit einem heterocyclischen Amid bzw. Thioamid der Formel III,

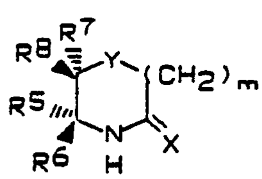

III

oder daß man
c) eine Verbindung der Formel II,

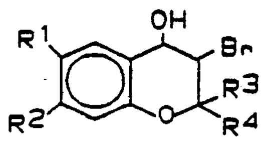

II,

in der $R^1$, $R^2$, $R^3$ und $R^4$ die obengenannten Bedeutungen haben, umsetzt mit einer Verbindung der Formel V

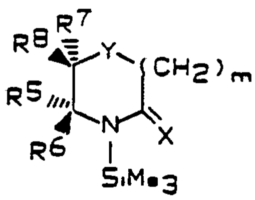

V

in der $R^5$, $R^6$, $R^7$, $R^8$, X, Y und m die obengenannten Bedeutungen haben,
oder daß man

d) eine Verbindung der Formel IV

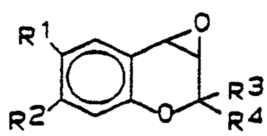

IV

in der $R^1$, $R^2$, $R^3$ und $R^4$ die obigen Bedeutungen aufweisen, mit einer Verbindung der Formel V,

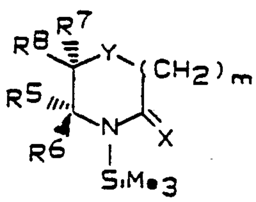

V,

in der $R^5$, $R^6$, $R^7$, $R^8$, X, Y und m die obengenannten Bedeutungen haben, zur Reaktion bringt,
oder daß man

e) eine Verbindung der Formel VI,

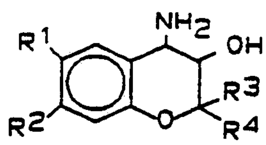

VI

in der $R^1$, $R^2$, $R^3$ und $R^4$ die obengenannten Bedeutungen haben, acyliert zur Verbindung der Formel VII,

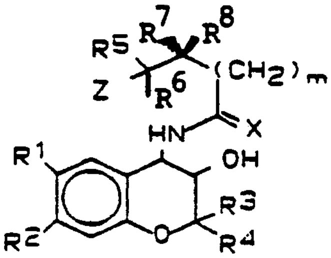

VII

in der $R^1$, $R^2$, $R^3$, $R^4$, $R^5$, $R^6$, $R^7$, $R^8$, X und m wie oben definiert sind und Z die Bedeutung einer

Fluchtgruppe wie Chlor oder Brom hat,
und diese cyclisiert zu einer Verbindung der Formel I.

8. Verbindung I nach Anspruch 1 zur Anwendung als Antihypertensivum und/oder Spasmolytikum.

9. Verfahren zum Herstellen eines Antihypertensivums, bzw. eines Spasmolytikums, dadurch gekennzeichnet, daß man eine wirksame Menge einer Verbindung I nach Anspruch 1 mit den pharmazeutisch üblichen Zusatzstoffen versieht.

10. Verfahren zum Behandeln des Bluthochdrucks und zur Spasmolyse, dadurch gekennzeichnet, daß man eine wirksame Verbindung I nach Anspruch 1 dem zu behandelnden Lebewesen verabreicht.

Patentansprüche für folgende Vertragsstaaten: ES, GR

1.Ein Verfahren zum Herstellen einer Verbindung I

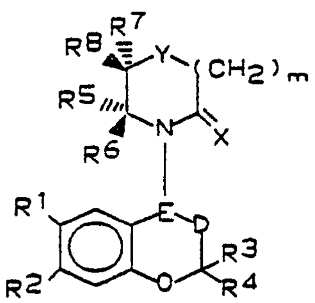

I ,

in welcher
E - D für
a) CH - CH(OH) oder
b) C = CH steht,
X für O oder S,
Y O, S, SO, $SO_2$ und $NR^9$ steht, wobei $R^9$ für Wasserstoff, $(C_1-C_6)$-Alkyl, Phenyl- und Benzyl steht, wobei der Phenylring unsubstituiert oder durch ein oder zwei gleiche oder verschiedene Reste aus der Reihe $(C_1-C_2)$-Alkyl, $(C_1-C_2)$-Alkoxy, F, Cl, Br, Trifluormethyl, CN, $NO_2$, CO-$(C_1-C_2)$-Alkyl substituiert ist, bedeutet,
$R^1$ für CN, $NO_2$, F, Cl, Br, $CF_3$, $(C_1-C_6)$-Alkoxycarbonyl, $SO_n$-$(C_1-C_6)$-Alkyl, $SO_n$-Ar, steht; wobei n = 1 oder 2, Ar für ein aromatisches oder heteroaromatisches System steht, das unsubstituiert oder durch 1 bis 3 gleiche oder verschiedene Reste $(C_1-C_2)$-Alkyl, $(C_1-C_2)$-Alkoxy, F, Cl, Br, Trifluormethyl, CN, $NO_2$, CO-$(C_1-C_2)$-Alkyl, $SO_p$-$(C_1-C_2)$-Alkyl, substituiert ist und p für 1 oder 2 steht,
$R^2$ für H, OH, $(C_1-C_2)$-Alkoxy, $(C_1-C_2)$-Alkyl, F, Cl, Br oder $NR^{10}$ $R^{11}$ steht, wobei $R^{10}$ und $R^{11}$ gleich oder verschieden sind und für H, $(C_1-C_5)$-Alkyl oder $(C_1-C_5)$-Alkylcarbonyl stehen,
$R^3$ und $R^4$ gleich oder verschieden sind und für Alkyl mit 1-4 C-Atomen stehen,
die oben genannten Bedeutungen von $R^1$ und $R^2$ auch vertauscht sein können,
$R^5$ und $R^6$ gleich oder verschieden sind und für Wasserstoff $(C_1-C_8)$-n-Alkyl, $(C_1-C_{10})$iso-Alkyl-, $(CH_2)_{1-6}CO_2(C_1-C_6)$-Alkyl, $-(CH_2)_{1-6}CONR^{10}R^{11}$,
wobei $R^{10}$ und $R^{11}$ in vorstehenden Bedeutungen stehen,
$-(CH_2)_{0-6}CO_2H$, $-(CH_2)_{1-6}$-CO-$(C_1-C_6)$-Alkyl, $-CO_2$-$(C_1-C_6)$-Alkyl, $(C_1-C_6)$-Alkylmercapto-$(C_1-C_6)$alkyl-, $(C_1-C_6)$-Alkylsulfinyl-$(C_1-C_6)$alkyl-, $(C_1-C_6)$-Alkylsulfonyl-$(C_1-C_6)$alkyl-,$(C_1-C_7)$-Hydroxyalkyl, $(C_1-C_7)$-Mercaptoalkyl-, Amino-$(C_1-C_7)$-alkyl-, N-$(C_1-C_4)$-Alkylamino-$(C_1-C_7)$-alkyl-, N,N-Di$(C_1-C_4)$-alkylamino$(C_1-C_7)$-alkyl-, $(CH_2)_f$Ar steht,
wobei Ar ein aromatisches oder heteroaromatisches System bedeutet, das unsubstituiert oder durch 1 bis 3 gleiche oder verschiedene Reste aus der Reihe $(C_1-C_2)$-Alkyl, Hydroxy, $(C_1-C_2)$-Alkoxy, F, Cl, Trifluormethyl, CN, $NO_2$, CO-$(C_1-C_2)$-Alkyl, $CO_2$-$(C_1-C_2)$-Alkyl, $CO_2H$, $SO_p$-$(C_1-C_2)$Alkyl, substituiert ist,
p für 1 oder 2 steht, und
f null, 1, 2 und 3 bedeutet,
wobei jedoch Verbindungen ausgeschlossen sind, in denen $R^5$ und $R^6$ gleichzeitg Wasserstoff bedeuten,
$R^7$ und $R^8$ gleich oder verschieden sind und für Wasserstoff, $(C_1-C_3)$-Alkyl oder Phenyl stehen,
m null, 1 oder 2 bedeutet; ausgeschlossen sind jedoch Verbindungen, in denen gleichzeitig

1. m = 1

2. einer der beiden Substituenten $R^5$ oder $R^6$ gleich Methyl und der andere Wasserstoff, $R^7$ und $R^8$ Wasserstoff und

3. Y Sauerstoff, Schwefel, NH oder N-($C_1$-$C_4$)-Alkyl bedeuten, dadurch gekennzeichnet, daß man
a) eine Verbindung der Formel II

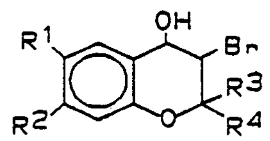

II,

in der $R^1$, $R^2$, $R^3$ und $R^4$ die obengenannten Bedeutungen haben, umsetzt mit einem Lactam bzw. Thiolaktam der Formel III

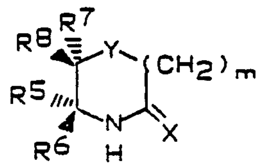

III,

in der $R^5$, $R^6$, $R^7$, $R^8$, X, Y und m die obengenannten Bedeutungen haben, oder daß man eine
b) Verbindung der Formel IV,

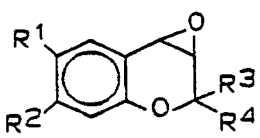

IV

in der $R^1$, $R^2$, $R^3$ und $R^4$ die oben genannten Bedeutungen haben, umsetzt mit einem heterocyclischen Amid bzw. Thioamid der Formel III,

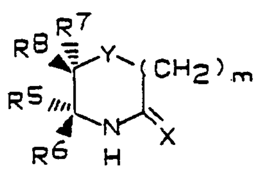

III

oder daß man
c) eine Verbindung der Formel II,

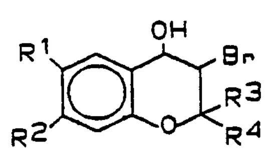

II,

in der $R^1$, $R^2$, $R^3$ und $R^4$ die obengenannten Bedeutungen haben, umsetzt mit einer Verbindung der Formel V

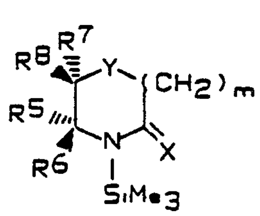

V

in der $R^5$, $R^6$, $R^7$, $R^8$, X, Y und m die obengenannten Bedeutungen haben,

oder daß man

d) eine Verbindung der Formel IV

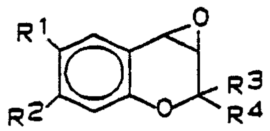

IV

in der $R^1$, $R^2$, $R^3$ und $R^4$ die obigen Bedeutungen aufweisen, mit einer Verbindung der Formel V,

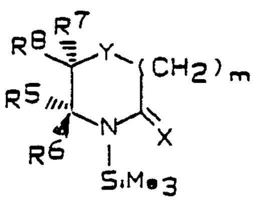

V,

in der $R^5$, $R^6$, $R^7$, $R^8$, X, Y und m die obengenannten Bedeutungen haben, zur Reaktion bringt, oder daß man

e) eine Verbindung der Formel VI,

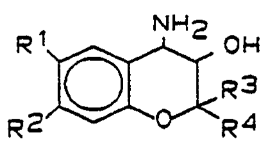

VI

in der $R^1$, $R^2$, $R^3$ und $R^4$ die obengenannten Bedeutungen haben, acyliert zur Verbindung der Formel VII,

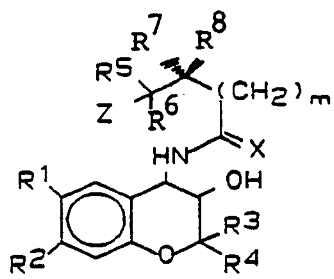

VII

in der $R^1$, $R^2$, $R^3$, $R^4$, $R^5$, $R^6$, $R^7$, $R^8$, X und m wie oben definiert sind und Z die Bedeutung einer Fluchtgruppe wie Chlor oder Brom hat,

und diese cyclisiert zu einer Verbindung der Formel I.

2. Verfahren nach Anspruch 1 dadurch gekennzeichnet, daß die folgenden Substituenten und Indices folgende Bedeutungen haben:

$R^1$, $R^2$, $R^3$, $R^4$ wie in Anspruch 1 definiert, einer der beiden Substituenten $R^5$ oder $R^6$ ist Wasserstoff oder Methyl und der andere wie in Anspruch 1 definiert,

$R^7$, $R^8$, $R^9$, $R^{10}$, $R^{11}$, m, f, X und Y wie in Anspruch definiert.

3. Verfahren nach Anspruch 1, dadurch gekennzeichnet, daß die folgenden Substituenten und Indices die folgenden Bedeutungen haben:

$R^1$ für CN, $NO_2$, $SO_2$-($C_1$-$C_4$)-Alkyl, ($C_1$-$C_4$)-Alkoxycarbonyl, F, Cl, $CF_3$,

$R^2$ für H und ($C_1$-$C_2$)-Alkoxy,

$R^3$ und $R^4$ für Methyl und Ethyl,

einer der beiden Substiuenten $R^5$ und $R^6$ für Wasserstoff oder Methyl und der andere für ($C_1$-$C_8$)-n-Alkyl, ($C_1$-$C_{10}$)-iso-Alkyl, $(CH_2)_{1-6}CO_2$($C_1$-$C_6$)-Alkyl, -$(CH_2)_{1-6}CONR^{10}R^{11}$, wobei $R^{10}$ und $R^{11}$ in obigen Bedeutungen stehen,

-$(CH_2)_{1-6}$-$CO_2H$, -$(CH_2)_{1-6}$-CO-($C_1$-$C_6$)-Alkyl, -$CO_2H$, ($C_1$-$C_6$)-Alkylmercapto-($C_1$-$C_6$)alkyl-, ($C_1$-$C_6$)-Alkylsulfinyl-($C_1$-$C_6$)alkyl-, ($C_1$-$C_6$)-Alkylsulfonyl- ($C_1$-$C_6$)alkyl-,($C_1$-$C_7$)-Hydroxyalkyl, ($C_1$-$C_7$)-Mercaptoalkyl-,

Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,
wobei Ar ein aromatisches oder heteroaromatisches System bedeutet, das unsubstituiert oder durch 1 bis 3 gleiche oder verschiedene Rest aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_p$-($C_1$-$C_2$)Alkyl, substituiert ist,
p für 1 oder 2 steht, und
f null, 1, 2 und 3 bedeutet,
$R^7$ und $R^8$ gleich oder verschieden sind und für Wasserstoff ($C_1$-$C_3$)-Alkyl und Phenyl stehen,
$R^9$ für H, ($C_1$-$C_4$)-Alkyl, Phenyl und Benzyl steht, wobei der Phenylring unsubstituiert oder durch ein oder zwei gleiche oder verschiedene Reste aus der Reihe ($C_1$-$C_2$)-Alkyl, ($C_1$-$C_2$)-Alkoxy, F, Cl, Trifluormethyl, CN, $NO_2$, CO-($C_1$-$C_2$)-Alkyl substituiert ist
m = null oder 1 bedeutet,
X für O oder S und
Y für O, SO, $SO_2$ oder $NR^9$ steht, worin $R^9$ wie oben definiert ist.

4. Verfahren nach Anspruch 1, dadurch gekennzeichnet, daß die folgenden Substituenten und Indices die folgenden Bedeutungen haben:
$R^1$ für $SO_2$-Phenyl steht, wobei Phenyl unsubstituiert oder durch 1 bis 3 Substituenten aus der Reihe ($C_1$-$C_2$)-Alkyl, ($C_1$-$C_2$)-Alkoxy, F, Cl, Br, Trifluormethyl, CN, $NO_2$, CO-($C_1$-$C_2$)-Alkyl, $SO_p$-($C_1$-$C_2$)-Alkyl, substituiert ist und p für 1 oder 2 steht,
$R^2$ für H und ($C_1$-$C_2$)-Alkoxy steht,
$R^3$ und $R^4$ für Methyl und Ethyl steht,
einer der beiden Substiuenten $R^5$ und $R^6$ für Wasserstoff oder Methyl und der andere für ($C_1$-$C_8$)-n-Alkyl, ($C_1$-$C_{10}$)-iso-Alkyl, $-(CH_2)_{1-6}CO_2$($C_1$-$C_6$)-Alkyl, $-(CH_2)_{1-6}CONR^{10}R^{11}$, wobei $R^{10}$ und $R^{11}$ in obigen Bedeutungen stehen,
$-(CH_2)_{1-6}$-$CO_2H$, $-(CH_2)_{1-6}$-CO-($C_1$-$C_6$)-Alkyl, -$CO_2H$, ($C_1$-$C_6$)-Alkylmercapto-($C_1$-$C_6$)alkyl-, ($C_1$-$C_6$)-Alkylsulfinyl-($C_1$-$C_6$)alkyl-, ($C_1$-$C_6$)-Alkylsulfonyl- ($C_1$-$C_6$)alkyl-, ($C_1$-$C_7$)-Hydroxyalkyl, ($C_1$-$C_7$)-Mercaptoalkyl-, Amino-($C_1$-$C_7$)-alkyl-, N-($C_1$-$C_4$)-Alkylamino-($C_1$-$C_7$)-alkyl-, N,N-Di($C_1$-$C_4$)-alkylamino($C_1$-$C_7$)-alkyl-, $(CH_2)_f$Ar steht,
wobei Ar ein aromatisches oder heteroaromatisches System bedeutet, das unsubstituiert oder durch 1 bis 3 gleiche oder verschiedene Reste aus der Reihe ($C_1$-$C_2$)-Alkyl, Hydroxy, ($C_1$-$C_2$)-Alkoxy, F, Cl, Trifluormethyl, CN, $NO_2$, CO-($C_1$-$C_2$)-Alkyl, $CO_2$-($C_1$-$C_2$)-Alkyl, $CO_2H$, $SO_p$-($C_1$-$C_2$)Alkyl, substituiert ist,
p für 1 oder 2 steht, und
f null, 1, 2 und 3 bedeutet,
$R^7$ und $R^8$ gleich oder verschieden sind und für Wasserstoff ($C_1$-$C_3$)-Alkyl und Phenyl stehen,
$R^9$ für H, ($C_1$-$C_4$)-Alkyl, Phenyl und Benzyl steht, wobei der Phenylring unsubstituiert oder durch einen oder zwei gleiche oder verschiedene Reste aus der Reihe ($C_1$-$C_2$)-Alkyl, ($C_1$-$C_2$)-Alkoxy, F, Cl, Trifluormethyl, CN, $NO_2$, CO-($C_1$-$C_2$)-Alkyl substituiert ist,
m = null oder 1 bedeutet,
X für O oder S und
Y für O, SO, $SO_2$ oder $NR^9$ mit $R^9$ in der oben angegebenen Bedeutung steht.

5. Verfahren nach Anspruch 1, dadurch gekennzeichnet, daß die folgenden Substituenten Indices die folgenden Bedeutungen haben:
E-D wie oben unter a) definiert und
$R^1$ für CN oder $SO_2CH_3$,
$R^2$ für H,
$R^3$ und $R^4$ für Methyl steht,
einer der Substituenten $R^5$ und $R^6$ Wasserstoff bedeutet und der andere für ($C_1$-$C_6$)-n-Alkyl, ($C_1$-$C_8$)-iso-Alkyl, Phenyl, Benzyl, Pyridyl steht, wobei die aromatischen bzw. heteroaromatischen Systeme unsubstituiert oder mit einem Rest aus der Reihe ($C_1$-$C_2$)-Alkyl, ($C_1$-$C_2$)-Alkoxy, Chlor, Fluor, $CF_3$ und CN substituiert sind, $R^7$ und $R^8$ für Wasserstoff steht,
m = null bedeutet,
X für O und
Y für O oder $NR^9$ steht, worin $R^9$ ($C_1$-$C_4$)-Alkyl und Benzyl bedeutet.

6. Verfahren zum Herstellen eines Antihypertensivums, oder eines Spasmolytikums, dadurch gekennzeichnet, daß man eine wirksame Menge einer Verbindung I nach Anspruch 1 mit den pharmazeutisch üblichen Zusatzstoffen versieht.

7. Verfahren zum Behandeln des Bluthochdrucks und zur Spasmolyse, dadurch gekennzeichnet, daß man eine wirksame Verbindung I nach Anspruch 1 dem zu behandelnden Lebewesen verabreicht.